# EUROPÄISCHE PATENTSCHRIFT

(11) **EP 1 819 654 B1**
(45) Veröffentlichungstag und Bekanntmachung des Hinweises auf die Patenterteilung: **10.06.2009**
(21) Anmeldenummer: 05810891.1
(22) Anmeldetag: 24.11.2005
(51) Int. Cl.: C07C 29/17, C07C 35/12, C07B 53/00

(54) **VERFAHREN ZUR HERSTELLUNG VON MENTHOL**
METHOD FOR THE PRODUCTION OF MENTHOL
PROCEDE DE PRODUCTION DE MENTHOL

(30) Priorität: 26.11.2004 DE 102004057277
(43) Veröffentlichungstag der Anmeldung: 22.08.2007
(73) Patentinhaber: BASF SE, 67056 Ludwigshafen (DE)
(72) Erfinder: BERGNER, Eike Johannes, 69198 Schriesheim (DE); EBEL, Klaus, 68623 Lampertheim (DE); JOHANN, Thorsten, 67117 Limburgerhof (DE); LÖBER, Oliver, 55234 Freimersheim (DE)
(86) Internationale Anmeldenummer: PCT/EP2005/012563
(87) Internationale Veröffentlichungsnummer: WO 2006/056435

(56) Entgegenhaltungen:
- EP-A- 1 053 974
- WO-A-20/05085160
- KURT BAUER, DOROTHEA GARBE, HORST SUBURG: "Common Fragrance and Flavor Materials: Preparation and Uses, Fourth, Completely Revised Edition; Chapter 2: Single Fragrance and Flavor Materials" 2001, WILEY-VCH VERLAG GMBH , XP002364464 Seite 26, Zeile 5 Seite 27, Zeilen 15,16,31,32 Seite 32, Zeilen 3,23-27 Seite 39, Zeilen 9,10,20-22,27 Seite 53, Absatz 6 - Seite 54, Absatz 2 Seite 56, Absätze 3,4
- NOYORI R.: 'Asymmetric Catalysis: Science and Opportunities (Nobel Lecture)' ANGEW. CHEM. INT. ED. Bd. 41, 2002, Seiten 2008 - 2022

## Beschreibung

### Technisches Gebiet der Erfindung

Die vorliegende Erfindung betrifft ein Verfahren zur Herstellung von optisch aktivem Menthol ausgehend von Geraniol oder Nerol oder Gemischen von Geraniol und Nerol.

Menthol stellt eine der wichtigsten Aromachemikalien dar, deren Hauptmenge nach wie vor aus natürlichen Quellen isoliert wird. Für totalsynthetische Zugänge zum Menthol, insbesondere dem natürlich vorkommenden Enantiomeren L-Menthol in technischem Maßstab besteht daher ständiger Optimierungsbedarf bezüglich der Wirtschaftlichkeit des Verfahrens. Insbesondere die Synthese von L-Menthol ausgehend von wohlfeilen achiralen Edukten stellt deshalb nach wie vor eine Herausforderung dar.

Zur Synthese von L-Menthol können zwei Strategien verfolgte werden: Zum einen lässt sich racemisches Menthol, das beispielsweise durch Hydrierung von Thymol zugänglich ist, nach Veresterung durch Racematspaltung (durch Kristallisation oder enzymatische resolution) erhalten, wie beispielsweise in der EP-A 0 743 295, der EP-A 0 563611 oder der EP-A 1 223 223 beschrieben. Andererseits lassen sich auch asymmetrische Synthesestrategien verfolgen, die einen enantioselektiven Syntheseschritt beinhalten.

### Stand der Technik

K. Tani et al. beschreiben in J. Chem. Soc. Chem. Comm. 1982, 11, 600-601 die asymmetrische Synthese von L-Menthol ausgehend von Diethylgeranylamin. Dabei wird Diethylgeranylamin in Gegenwart eines kationischen Rh(I)-Komplexes als Katalysator zu dem entsprechenden optisch aktiven Enamin isomerisiert.

Die JP-A 53-116348 offenbart die Herstellung von L-Isopulegol, einem Vorläfer des Menthols, durch Cyclisierung von L-Isopulegol durch selektive Cyclisierung von D-Citronellal in Gegenwart von Zinkbromid als Katalysator.

S. Akutagawa beschreibt in Topics in Catalysis 4 (1997) 271- 274 die Synthese von L-Menthol durch enatioselektive Isomerisierung von Allylaminen zu Enaminen mit Rh-BINAP (2,2'-Bis(diphenylphoshino)-1,1'-binaphtyl) als Katalysator.

R. Noyori et al. beschreiben in J. Am. Chem. Soc. 1987, 109, 1596-1597 und in Organic Synthses 1995, 72, 74-85 die asymmetrische Hydrierung von Geraniol mittels eines Ru-BINAP Katalysators.

Die US 6,342,644 offenbart ein Verfahren zur asymmetrischen Synthese von L-Menthol durch enantioselektive Hydrierung von Piperitenon als Schlüsselschritt.

R. NOYORI offenbart in Angew. Chem. Int. Ed., Bd. 41, 2002, Seiten 2008-2022 einen 5-stufigen Prozess umfassend die Schritte a) regio- und stereoselektive Addition von Lithium-Diethylamid an Myrcen unter Erhalt von N, N-Diethylgeranyl-amin, b) asymmetrische Isomerisierung des N,N-Diethylgeranyl-amins in Gegenwart von Rhodium (S)-BINAP zum (R)-Citronellyl-enamin, c) saure Hydrolyse des (R)-Citronellyl-enamins zum (R)-Citronellal,d) Cyclisierung in Gegenwart von ZnBr2 als Lewis-Säure zum L-Isopulegol und e) katalytische Hydrierung zum L-Menthol.

### Aufgabe der Erfindung

Der vorliegenden Erfindung lag die Aufgabe zu Grunde, ein Verfahren zur asymmetrische Synthese von optisch aktivem Menthol ausgehend von einem wohlfeilen, achiralen und seinerseits gut synthetisch zugänglichem, d.h. nicht notwendigerweise aus natürlichen Quellen zu isolierenden Ausgangsstoff bzw. Ausgangstoffen bereitzustellen.

### Beschreibung der Erfindung sowie der bevorzugten Ausführungsformen

Die Aufgabe wurde erfindungsgemäß gelöst durch die Bereitstellung eines Verfahrens zur Herstellung von optisch aktivem Menthol ausgehend von Geraniol oder Nerol oder Gemischen von Geraniol und Nerol indem man
a) Geraniol oder Nerol oder Gemische von Geraniol und Nerol zu optisch aktivem Citronellol enantioselektiv hydriert,
b) das so erhaltene optisch aktive Citronellol zu optisch aktivem Citronellal umsetzt,
c) das so erhaltene optisch aktive Citronellal zu einem optisch aktives Isopulegol enthaltenden Stoffgemisch cyclisiert und
d) optisch aktives Isopulegol aus dem so erhaltenen Stoffgemisch abtrennt und zu optisch aktivem Menthol hydriert oder das in dem so erhaltenen Stoffgemisch enthaltene optisch aktive Isopulegol zu optisch aktivem Menthol hydriert und das so erhaltene optisch aktive Menthol aus dem als Produkt der Hydrierung erhaltenen Stoffgemisch abtrennt.

Als Ausgangssubstanz bzw. Ausgangssubstanzen zur Durchführung des erfindungsgemäßen Verfahren dienen die Verbindungen Geraniol der Formel (I) und/oder Nerol der Formel (II) 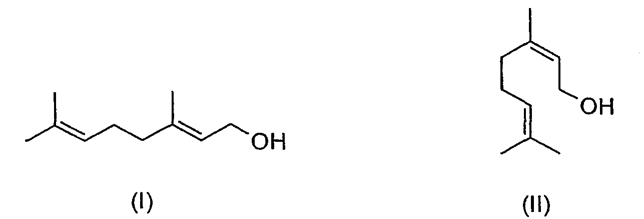 jeweils als solche oder in Form von Gemischen derselben. Dabei können die einzusetzenden einzelnen Verbindungen oder die einzusetzenden Gemische derselben noch geringe Mengen, üblicherweise bis zu etwa 5 Gew.-%, bevorzugt bis zu etwa 3 Gew.-% weiterer Verbindungen bzw. Verunreinigungen wie beispielsweise Lösemittelrückstände, Wasser oder Nebenprodukte vorausgeschalteter Syntheseschritte enthalten.

Ein bevorzugter Ausgangsstoff ist Geraniol, insbesondere solches mit einer Reinheit von mindestens 95 Gew.-%, das als Verunreinigung zur Hauptmenge Nerol enthält. Typischerweise setzt man Geraniol ein, das zu etwa 0,1 bis etwa 5 Gew.-% Nerol enthält. Solche Gemische der genannten Verbindungen fallen beispielsweise bei der technischen Synthese von Geraniol durch selektive Hydrierung von Citral und anschließender destillativer Aufreinigung an, wie sie beispielsweise in der EP- A 1 317 959, EP-A 1 318 129, DE-A 31 38 423 oder der DE-A 101 60 143 beschrieben ist.

In einer weiteren Ausführungsform des erfindungsgemäßen Verfahrens lassen sich auch dementsprechende Gemische von Nerol, d.h. Nerol das zu etwa 0.1 bis etwa 10 Gew.-% Geraniol enthält oder auch reines Nerol als Ausgangsstoff einsetzen.

### Beschreibung Schritt a): Enantioselektive Hydrierung

Die genannten Ausgangsstoffe, bevorzugt Geraniol, werden in einem ersten Schritt des erfindungsgemäßen Verfahrens zu optisch aktivem Citronellol der Formel (III) enantioselektiv hydriert.

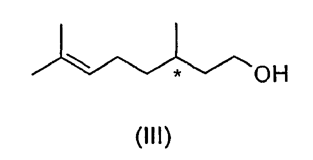

Unter dem Begriff optisch aktives Citronellol ist dabei solches Citronellol zu verstehen, das zum überwiegenden Teil aus einem der beiden möglichen Enantiomeren des Citronellols besteht. Bevorzugt ist unter optisch aktivem Citronellol im Rahmen der vorliegenden Erfindung solches Citronellol zu verstehen, das einen Enantiomerenüberschuss (ee) von mindestens 90% ee, bevorzugt von etwa 95 bis etwa 99% ee aufweist. Dabei kann in Abhängigkeit von den gewählten Reaktionsbedingungen, insbesondere in Abhängigkeit des eingesetzten Katalysators, das mit (*) in Formel (III) gekennzeichnete asymmetrische Kohlenstoffatom entweder in der R- oder der S-Konfiguration vorliegen.

Die enantioselektive Hydrierung führt man in dem Fachmann bekannter Weise in Gegenwart eines geeigneten Katalysators sowie in Gegenwart von Wasserstoff durch. Als geeignete Katalysatoren sind solche zu betrachten, die in der Lage sind, dreifach substituierte ethylenische Doppelbindungen, insbesondere solche in Nachbarschaft einer Hydroxylgruppe, enantioselektiv zu hydrieren.

Bevorzugt führt man die genannte enantioselektive Hydrierung gemäß Schritt a) in Gegenwart eines homogenen Übergangsmetallkatalysators durch, der Ru, Rh oder Ir sowie einen chiralen, mindestens ein Phosphor-, Arsen- und/oder Antimon-Atom, bevorzugt mindestens ein Phosphor-Atom aufweisenden Liganden umfasst. Derartige Katalysatoren sind, wie Eingangs erwähnt, bekannt und beispielsweise in T. Ohkuma et al. Asymmetric Synth. (2nd Ed.) 1999, 1-110; Dep. Chem., Res. Cent. Mater. Sci., Nagoya 464, Japan oder W. Tang et al. Chem. Rev. 2003, 103, 3029-3069 beschrieben.

Insbesondere bevorzugt als Liganden sind phosphorhaltige Verbindungen mit der Fähigkeit, Atropisomerie bezüglich zweier Aryl- bzw. Hetarylsysteme auszubilden gemäß der folgenden allgemeinen Formeln (IV) bis (VI): 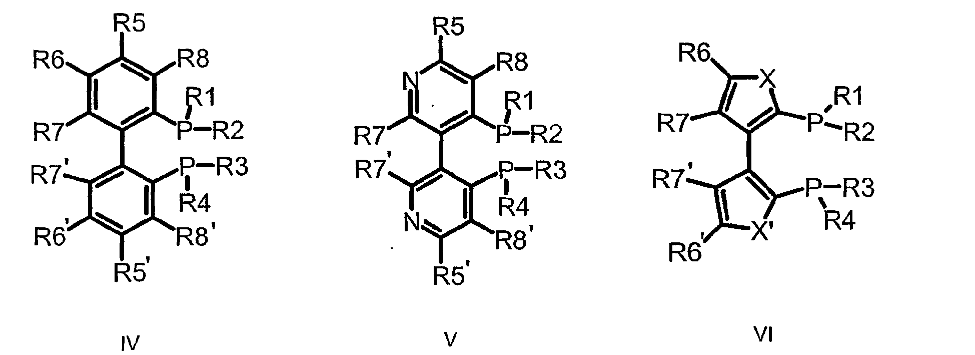 wobei die Reste R1 bis R10, R5' bis R8' sowie X und X' folgende Bedeutungen besitzen:
- R1, R2: können gleich oder verschieden sein und durch Halogen, C₁- bis C₆-Alkyl oder C₁- bis C₆-Alkoxy substituiertes oder unsubstituiertes Aryl, Heteroaryl, Alkyl, Cycloalkyl mit 1 bis 20 Kohlenstoffatomen bedeuten;
- R3, R4: können gleich oder verschieden sein und durch Halogen, C₁- bis C₆-Alkyl oder C₁- bis C₆-Alkoxy substituiertes oder unsubstituiertes Aryl, Heteroaryl, Alkyl, Cycloalkyl mit 1 bis 20 Kohlenstoffatomen bedeuten;
- R5, R5': können gleich oder verschieden sein und Wasserstoff, Halogen, C₁- bis C₆-Alkyl, C₆- bis C₁₀-Aryl, C₁- bis C₆-Alkoxy, Amino oder Thio bedeuten;
- R6, R6': können gleich oder verschieden sein und Wasserstoff, Halogen, C₁- bis C₆-Alkyl, C₆- bis C₁₀-Aryl, C₁- bis C₆-Alkoxy, Amino oder Thio bedeuten;
- R7, R7': können gleich oder verschieden sein und Wasserstoff, Halogen, C₁- bis C₆-Alkyl, C₆- bis C₁₀-Aryl, C₁₋ bis C₆-Alkoxy, Amino oder Thio bedeuten;
wobei R6 und R7 (falls beide vorhanden) und/oder R6' und R7' (falls beide vorhanden) jeweils gemeinsam einen oder mehrere Ringe, die weitere 1 oder 2 Doppelbindungen und/oder eines oder mehrere Heteroatome ausgewählt aus der Gruppe der Heteroatome N, O, S enthalten können, bilden können;
- R8, R8': können gleich oder verschieden sein und Wasserstoff, Halogen, C₁- bis C₆-Alkyl, C₆- bis C₁₀-Aryl, C₁- bis C₆-Alkoxy, Amino oder Thio bedeuten;
- X, X': können gleich oder verschieden sein und S, O oder NR9 bedeuten, wobei
- R9: Wasserstoff, C₁- bis C₆-Alkyl, C₆- bis C₁₀-Aryl, C₁- bis C₆-Acyl oder SO₂R10 bedeuten, wobei

R10 C₆- bis C₁₀-Aryl, C₁- bis C₆-Alkyl, C₁- bis C₆-Fluoroalkyl, insbesondere CF₃ bedeutet und unter Halogen Fluor, Chlor, Brom oder Jod, insbesondere Fluor oder Chlor zu verstehen ist.

Unter dem Begriff C₁-C₆-Alkyl sind dabei geradkettige, verzweigte oder cyclische Alkylreste mit 1 bis 6 Kohlenstoffatomen wie beispielsweise Methyl, Ethyl, Propyl, Isopropyl, n-Butyl, Isobutyl, tert.-Butyl, n-Pentyl, Isopentyl, Neopentyl, Cyclopentyl, n-Hexyl, Cyclohexyl zu verstehen.

Unter C₁- bis C₆-Alkoxy sind geradkettige oder verzweigte Alkoxysubstituenten mit 1 bis 6 Kohlenstoffatomen zu verstehen, wie beispielsweise Methoxy, Ethoxy, Propoxy, Isopropoxy.

C6- bis C10-Aryl bedeuten Phenyl oder Naphtyl; unter Heteroaryl ist insbesondere Pyridyl oder Thiophenyl zu verstehen.

Der Begriff Amino bedeutet -NH₂, -NHR11 oder -NR11 R12, wobei R11, R12 unabhängig voneinander jeweils C₁-C₆-Alkyl bedeuten können.

Unter dem Begriff Thio sind sowohl freie Thiolgruppen -SH als auch Thioether -SR11 zu verstehen.

Besonders gut geeignete Liganden für die in Schritt a) des erfindungsgemäßen Verfahrens bevorzugt zu verwendenden Katalysatorsysteme sind die folgenden aus der Literatur bekannten Liganden (1) bis (43) :

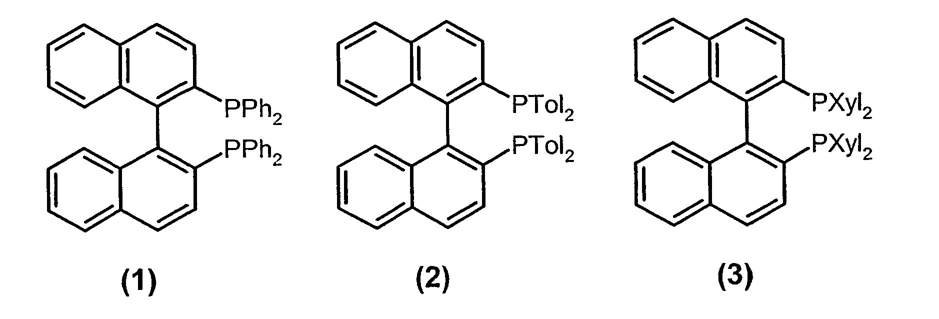

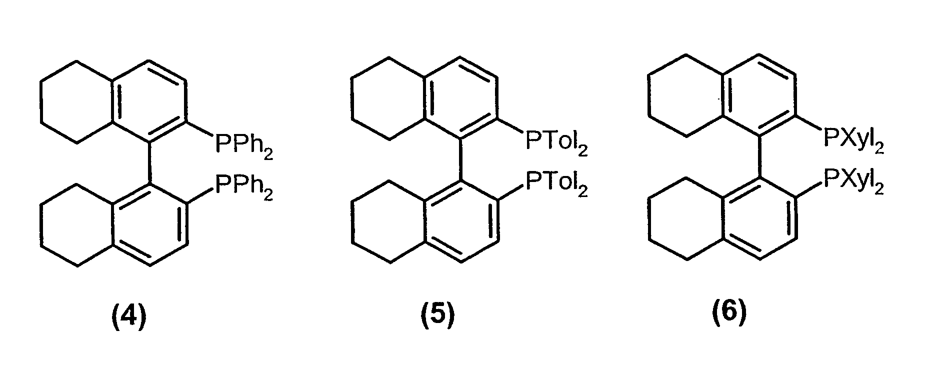

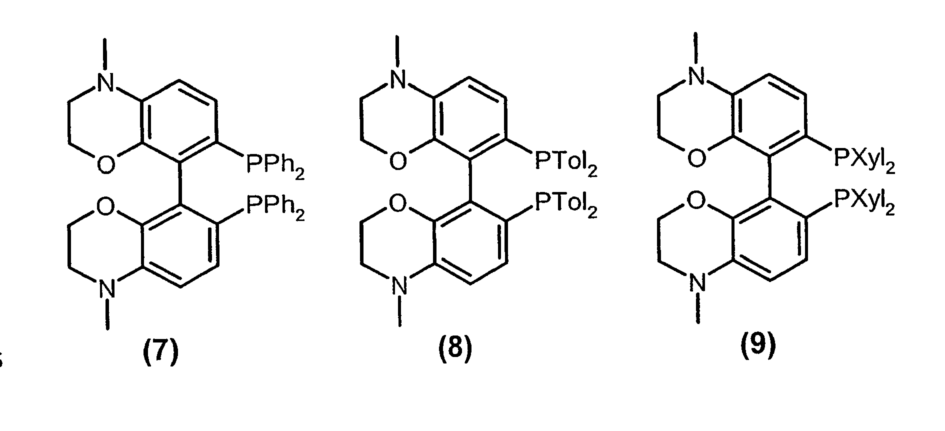

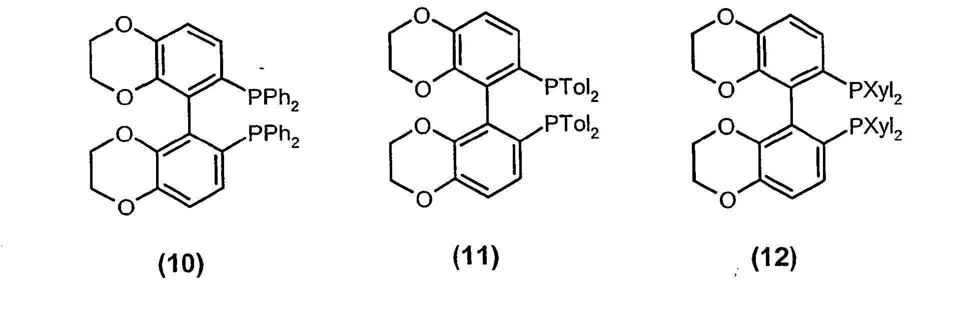

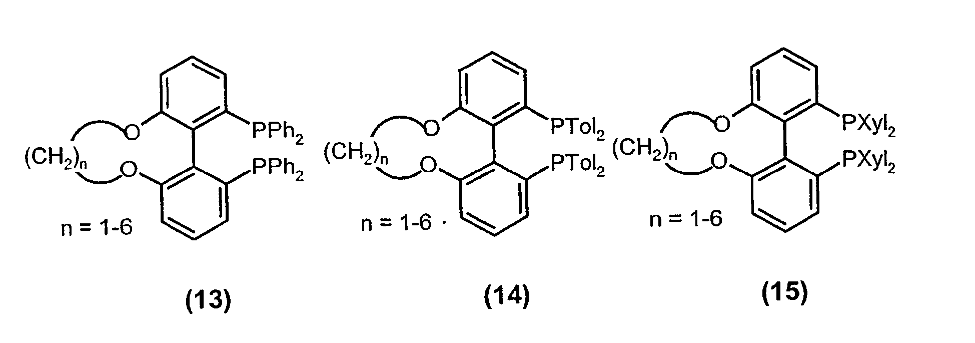

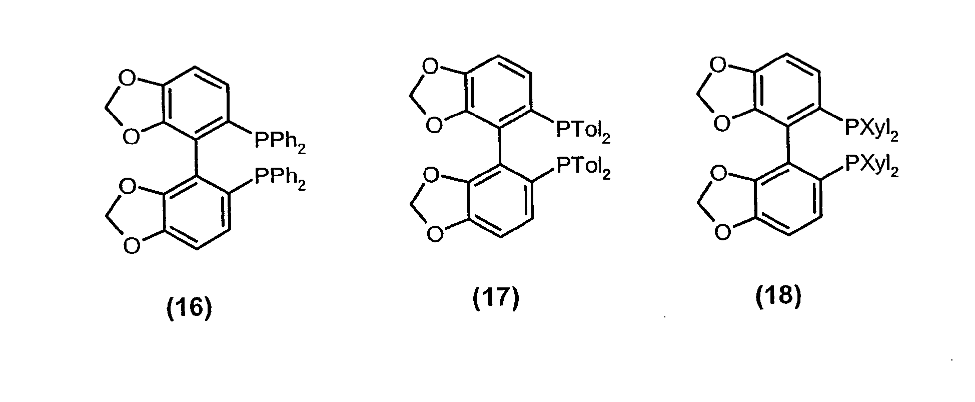

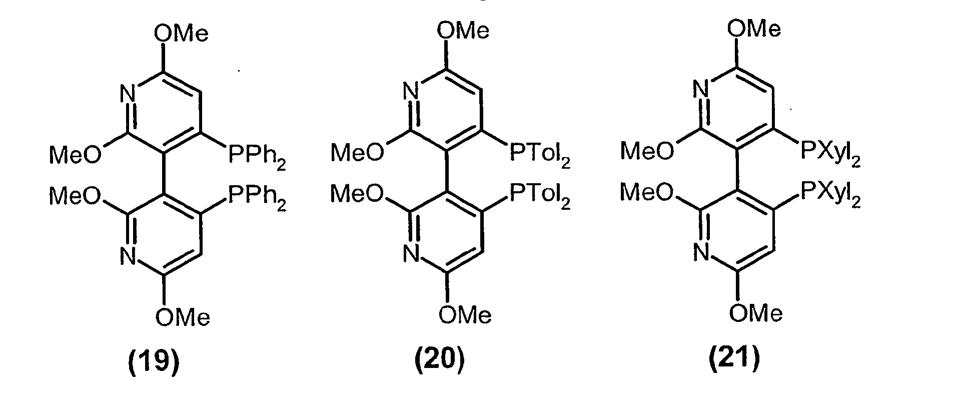

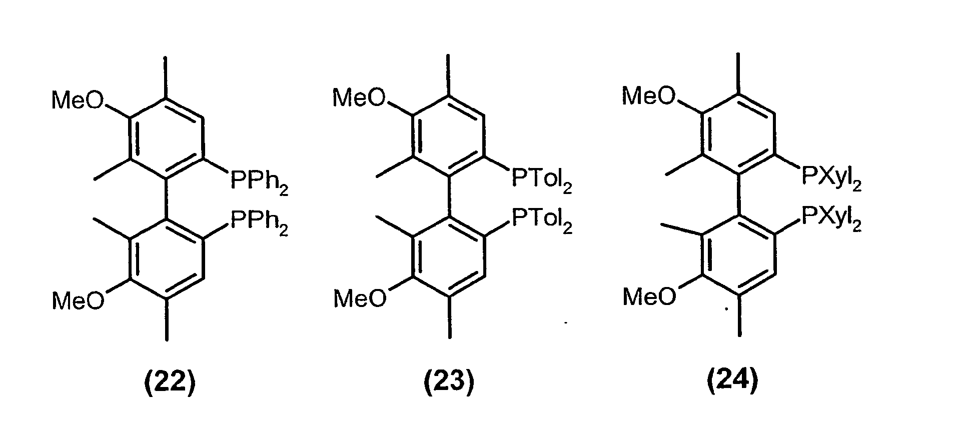

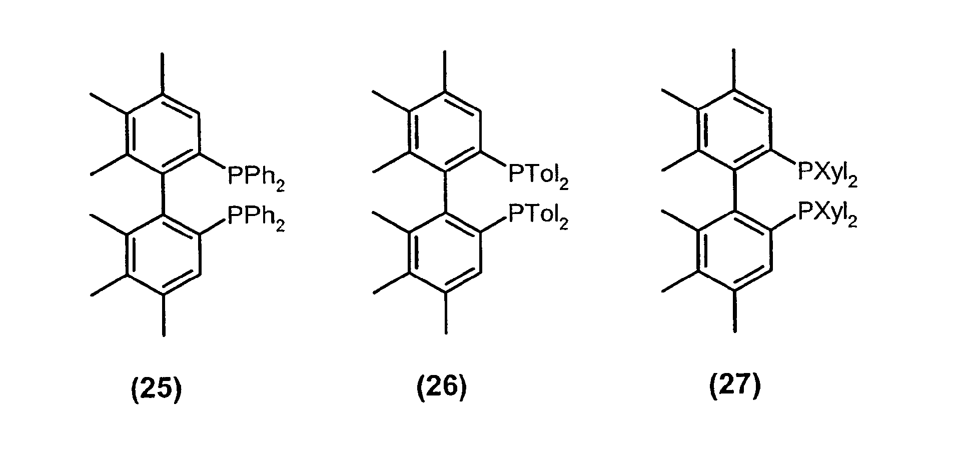

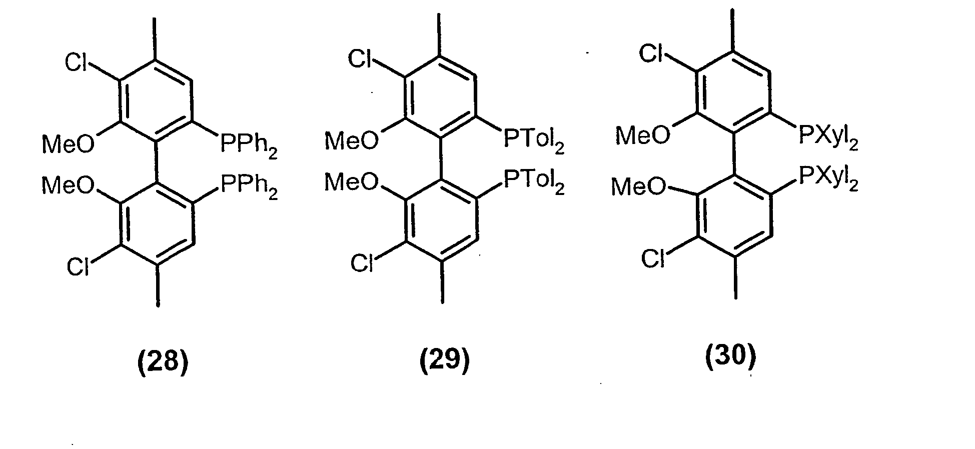

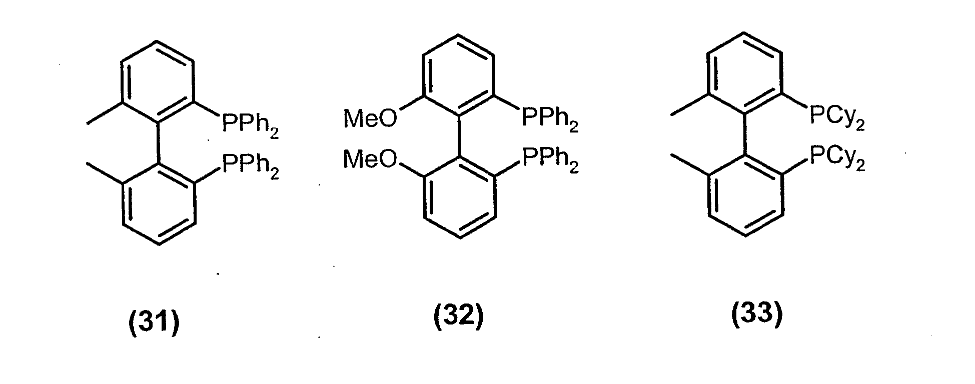

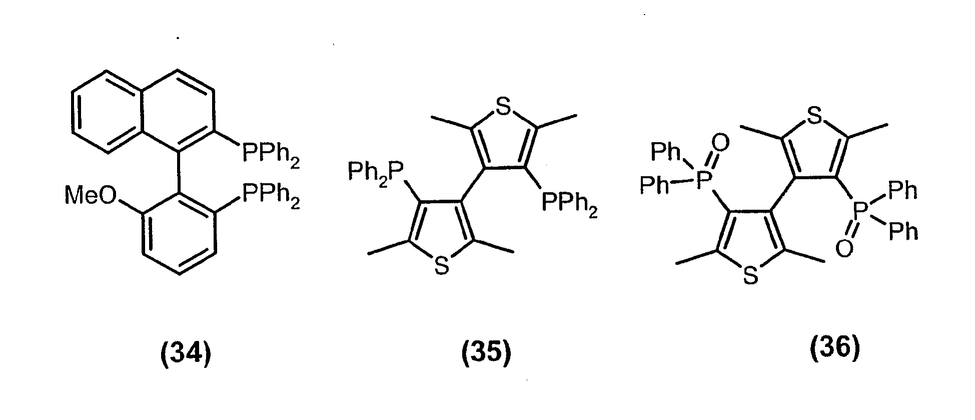

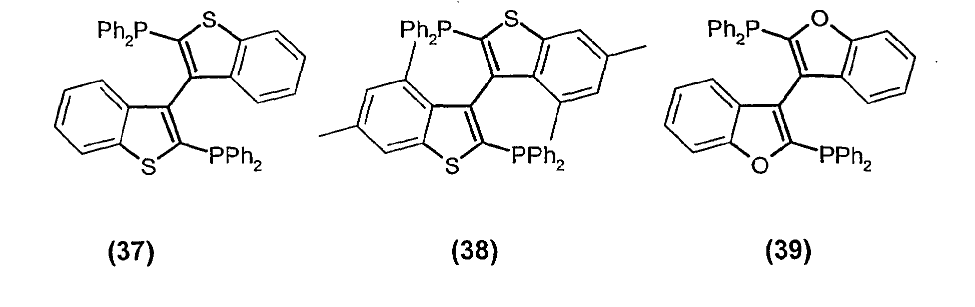

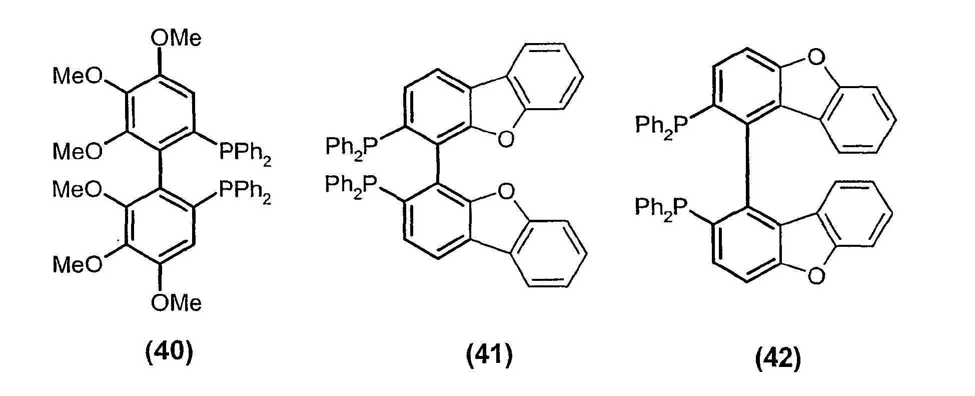

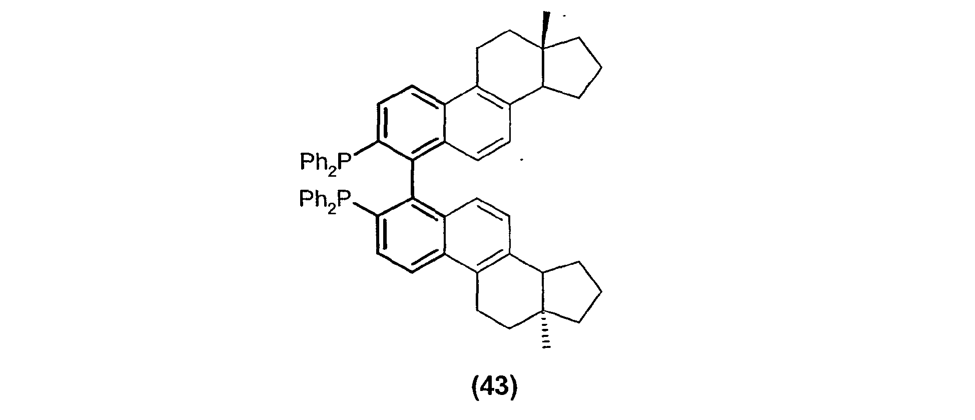

Dabei steht "Tol" für Tolyl, "Xyl" für Xylyl und "Cy" für Cyclohexyl.

Darüber hinaus kommen als Liganden auch die Aminophosphinliganden gemäß der folgenden allgemeinen Struktur (VII) in Betracht: 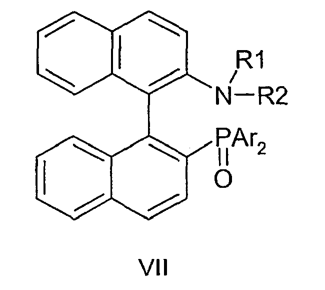 wobei
- R1, R2: die oben angegebene Bedeutung besitzen kann und
- Ar: C₆- bis C₁₀-Aryl oder -Heteroaryl bedeutet.

Die genannten Liganden werden dabei bevorzugt in enantiomerenreiner Form eingesetzt. Insbesondere bevorzugt ist ein Katalysator der Ru und einen Liganden, ausgewählt aus der Gruppe der Liganden 2,2'-Bis(diphenylphosphino-1,1'-binaphtyl (BINAP), 2,2'-Bis[di(p-tolyl)phosphino]-1,1'-binaphthyl (p-Tol-BINAP), 2,2'-Bis(diphenylphosphino)-4,4',6,6'-tetramethyl-3,3'-bibenzo[b]thiophen (tetraMetianp), 2,2'-Bis-(diphenylphosphino)-3,3'-tetramethyl-3,3'-bibenzo[b]thiophen (bitianp) enthält. Darüber hinaus bevorzugt ist ein Katalysator der Ru und BINAP (2,2'-Bis(diphenylphosphino- . 1,1'-binaphtyl) (Struktur (1)) oder p-Tol-BINAP (2,2'-Bis[di(p-tolyl)phosphino]-1,1'-binaphthyl) (Struktur (2)), insbesondere (S)-BINAP enthält.

Die genannten und weitere geeignete Übergangsmetallverbindungen und -komplexe sind bekannt und in der Literatur beschrieben oder können vom Fachmann analog zu den bereits bekannten Verbindungen hergestellt werden.

Wie dem Fachmann bekannt, können je nach Wahl der enantiomeren Formen der eingesetzten chiralen Liganden sowie in Abhängigkeit von der Konfiguration der zu hydrierenden Doppelbindung die beiden Enantiomere des Citronellals gezielt bevorzugt erhalten werden. So erhält man beispielsweise durch Hydrierung von Geraniol in Gegenwart eines Ru und (S)-BINAP enthaltenden Katalysators bevorzugt D-(R)-Citronellol.

Werden Iridium-Komplexe mit den vorstehend genannten Aminophosphin-Liganden, wie beispielsweise 2-Amino-2'-diarylphosphino-1-1'-binaphthyl (SMAP), 2-Amino-2'-diarylphosphinyl-1-1'-binaphthyl oder 2-Carbamoyl-2'-diarylphosphinyl-1-1'-binaphthyl für die asymmetrische Hydrierung von Geraniol bzw. Nerol eingesetzt, so kann sowohl mit reinem Nerol oder Geraniol oder sogar einem 1 : 1 Gemisch beider Terpenalkohole gearbeitet werden, wie in der EP-A 1 013 658 beschrieben.

Die genannten Katalysatoren können als fertige, teilweise kommerziell erhältliche Verbindungen eingesetzt werden oder in dem Fachmann an sich bekannter Weise zunächst aus Vorläuferverbindungen hergestellt werden. Geeignete Vorläuferverbindungen zur Herstellung von Ru-haltigen Komplexen sind beispielsweise [RuCl₂ (benzol)]₂, Ru(acac)₃, (RuCl₂(COD)], wobei unter acac Acetylacetonat und unter COD Cyclooctadien zu verstehen ist.

Üblicherweise werden die als Katalysatoren im Rahmen des erfindungsgemäßen Verfahrens einzusetzenden chiralen Rutheniumkomplexe in situ hergestellt, indem man die gewählte Vorläuferverbindung mit dem gewählten chiralen Liganden in einem geeigneten organischen Lösungsmittel und unter Ausschluss von Sauerstoff, d.h. vorzugsweise unter Inertgasatmosphäre, in Kontakt bringt. Dabei kann es von Vorteil sein, dem Gemisch Alkali- oder Erdalkalimetallsalze der Essigsäure bzw. der Trifluoressigsäure oder auch Essigsäure oder Trifluoressigsäure als solche zuzusetzen.

Das molare Verhältnis von eingesetztem Ruthenium (bezogen auf die in den Vorläuferverbindungen enthaltenen Ru-atome) zu eingesetztem chiralen Liganden beträgt üblicherweise etwa 1:1 1 bis etwa 2:1, bevorzugt etwa 1:1,05 bis etwa 1:1,5.

Als besonders geeignete Lösemittel zur in-situ Herstellung des Katalysators wie auch zur Durchführung der erfindungsgemäßen enantioselektiven Hydrierung haben sich Alkanole wie z.B. Methanol, Ethanol und/oder Isopropanol, aber auch Alkandiole wie z.B. Ethylenglycol, Dietylenglycol, Triethylenglycol und oder Tripropylenglycol erwiesen. Darüber hinaus kann die Hydrierung mit gutem erfolg auch ohne Zusatz von Lösemitteln, d.h. in der reinen Ausgangsverbindung, durchgeführt werden.

Insbesondere bevorzugt führt man die enantioselektive Hydrierung gemäß Schritt a) so durch, dass man Methanol als Lösemittel einsetzt. Bei der erfindungsgemäßen enantioselektiven Hydrierung von Geraniol zu Citronellol setzt man vorteilhaft eine 1 bis 50 Gew.%ige Lösung von Geraniol in Menthol ein. Bevorzugt setzt man eine 3 bis 20 Gew.-%ige, insbesondere bevorzugt eine 7 bis 12 Gew.-%ige Lösung von Geraniol in Methanol ein.

Die genannte Hydrierung wird üblicherweise in einem Temperaturbereich von 1 bis 60°C, bevorzugt von 20 bis 50°C und besonders bevorzugt von 35 bis 45°C und unter einem Wasserstoffdruck von 10 bis 150 bar, bevorzugt von 70 bis 120 bar durchgeführt. An die einzusetzenden Reaktionsgefäße bzw. Reaktoren sind keine besonderen Ansprüche zu stellen. Es eignen sich alle dem Fachmann zur Durchführung von Reaktionen unter erhöhtem Druck und erhöhter Temperatur geeignet erscheinenden Formen, die eine gute Durchmischung des Reaktionsgemisches gewährleisten.

Durch die genannten Maßnahmen lässt sich die erfindungsgemäße enantioselektive Hydrierung von Geraniol bzw. Nerol zur optisch aktivem Citronellal in besonders wirtschaftlicher Weise, insbesondere bei Umsetzungen in technischem Maßstab, durchführen. Je nach Wahl der Ausgangsverbindungen, Katalysatoren und Reaktionsbedingungen wurden Turnover-Numbers (TON) von bis zu 10000 erzielt.

Das wie vorstehend beschrieben erhaltene Citronellal kann nach allen dem Fachmann bekannten Methoden und Verfahren, insbesondere durch Destillation weiter aufgereinigt werden.

### Beschreibung Schritt b): Umsetzung zum optisch aktiven Citronellal

In einem zweiten Schritt b) des erfindungsgemäßen Verfahrens setzt man das wie vorstehend beschriebene, in Schritt a) erhaltene optisch aktive Citronellol zu optisch aktivem Citronellal der Formel (VIII) um.

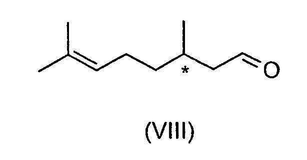

Dazu eignen sich alle Verfahren, Methoden bzw. Reaktionen (in Folgenden Transformationen) bei denen gewährleistet ist, dass der in der enantioselektiven Hydrierung gemäß Schritt a) erzielte Enantiomerenüberschuss (ee) des erhaltenen optisch aktiven Citronellols weitgehend erhalten bleibt. Demzufolge eignen sich zur Durchführung von Schritt b) des erfindungsgemäßen Verfahrens alle Transformationen, bei denen man Citronellal erhält, dessen Enantiomerenüberschuss (ee) mindestens 80%, bevorzugt mindestens 90%, insbesondere 95 bis 100% des Enantiomerenüberschusses des eingesetzten, in Schritt a) erhaltenen Citronellols entspricht.

Geeignete Reaktionen zur Bewerkstelligung der erfindungsgemäßen Oxidation bzw. Dehydrierung von optisch aktivem Citronellal zu optisch aktivem Citronellol sind beispielsweise die Oxidation mit Luftsauerstoff unter Fe(NO₃)₃-FeBr₃-Katalyse wie z.B. von S. E. Martin et al. in Tetrahedron Lett. 2003, 44, 549-552 bebschrieben; die TEMPO-Oxidation in ionischen Flüssigkeiten, wie beispielsweise von 1. A. Ansari et al. in Organic Letters, 2002, 4, 1507-1509 beschreiben; die Kupfer-katalysierte aerobe Oxidation mit TEMPO im zweiphasigen System mit fluorierten Lösungsmitteln, wie z.B. beschrieben von G. Ragagnin et al. in Tetrahedron, 2002, 58, 3985-3991; die Ruthenium-katalysierte aerobe Oxidation mit TEMPO, z.B. nach A. Dijksman et al., Chem. Commun., 1999, 1591-1591; die selektive Oxidation optisch aktiver Alkohole mit Dimethyldioxiran, wie z.B. von L. D'Accolti et al. in Org. Chem. 1998, 58, 3600-3601 beschrieben oder die selektive Oxidation von Alkoholen mit NaOCl und katalytischen mengen TPAP, wie von L. Gonsalvi et al in Organic Letters, 2002, 4, 1659-1661 beschrieben.

Ein im Rahmen der vorliegenden Erfindung bevorzugtes Verfahren zur Umsetzung von optisch aktivem Citronellol zu optisch aktivem Citronellal gemäß Schritt b) ist die Dehydrierung von optisch aktivem Citronellal in Gegenwart eines Katalysators in der Gasphase, wie sie in der internationalen Anmeldung mit dem Aktenzeichen PCT/EP/05/002288, auf die hiermit vollumfänglich Bezug genommen wird, beschrieben ist.

Zur Durchführung des erfindungsgemäß bevorzugten Dehydrierverfahrens gemäß Schritt b) eignet sich eine große Vielfalt von Katalysatoren, insbesondere solche Katalysatoren die mindestens eines der Elemente ausgewählt aus der Gruppe der Elemente Zink, Calzium und Kupfer, jeweils als solche oder in Form geeigneter Verbindungen enthalten.

Neben den genannten Elemente können die erfindungsgemäß in Schritt b) einsetzbaren Katalysatoren noch eines oder mehrere Elemente der Gruppen 1, 2, 3, 4, 13 und/oder 14, wie z.B. Na, K, Mg, Ti, Zr, C, Si, und/oder Ge enthalten.

Besonders geeignet zur Durchführung des bevorzugten Dehydrierverfahrens sind solche Katalysatoren, die Zink und Calcium enthalten, bevorzugt in oxidischer Form und/oder in Form ihrer Carbonate. Dabei sind insbesondere solche Katalysatoren zu bevorzugen, die Zinkoxid und Calciumcarbonat enthalten.

Bevorzugte Katalysatoren zur Durchführung des Dehydriererfahrens sind solche, deren aktive Komponente zu 30 bis 60 Gew.-%, bevorzugt zu 40 bis 50 Gew.-% aus Zinkoxid und zu 40 bis 70, bevorzugt zu 50 bis 60 Gew.-% aus Calciumcarbonaten bestehen. Weiterhin bevorzug sind darunter solche, deren Calciumcarbonat-Komponente in der Calcit-Modifikation vorliegt. Die genannten Mengenanteile sind aus der geglühten Katalysatormasse zu bestimmen, in der Zink und Calcium jeweils in Form ihrer Oxide vorliegen.

Weitere zur Dehydrierung von optisch aktivem Citronellol einsetzbare Katalysatoren sind Kupfer enthaltende Katalysatoren, insbesondere solche, die Kupfer in einer Oxidationsstufe >O in auf einem oxidischen Träger abgeschiedener Form enthalten, wie sie in der DE-A 197 57 297 beschrieben sind. Als weiteres Trägermaterial kommen beispielsweise auch Calciumcarbonat sowie weitere geeignete Trägermaterialien in Betracht.

In einer bevorzugten Ausführungsform weisen die erfindungsgemäß einsetzbaren Katalysatoren eine spezifische Oberfläche nach BET von 5 bis 50, vorzugsweise von 10 bis 30 m²/g auf.

Ein solcher Katalysator ist beispielsweise durch Ausfällen von schwerlöslichen Zink- und Calciumverbindungen aus wasserlöslichen Zink- und Calciumverbindungen mit einer Base und anschließender Aufarbeitung in an sich bekannter Weise erhältlich, wobei man
I. als Base ein wasserlösliches basisches Carbonat einsetzt,
II. gewünschtenfalls die schwerlöslichen Zink- und Calciumverbindungen nach dem Ausfällen filtriert,
III. die gewünschtenfalls filtrierten Zink- und Calciumverbindungen wäscht,
IV. die gewaschenen Zink- und Calciumverbindungen aus Schritt III. trocknet unter Erhalt eines Pulvers, und anschließend
V. das Pulver aus Schritt IV. bei Temperaturen von nicht über 600°C calciniert, und
VI. gewünschtenfalls das calcinierte Pulver zu Formkörpern verpresst.

Als wasserlösliche Zink- und Calciumsalze kann man Acetate, Sulfate, Nitrate, Chloride, bevorzugt Nitrate wie Zinknitrat, Zinkacetat, Zinksulfat, Calciumacetat, Calciumnitrat, bevorzugt Zinknitrat und Calciumnitrat, einsetzen. Üblicherweise setzt man wässrige Lösungen der entsprechenden Salze in Konzentrationen im Bereich von 3 bis 25, bevorzugt von 10 bis 25, insbesondere 20 Gew.%, ein.

Das Molverhältnis von Zink zu Calcium wählt man bevorzugt so, dass nach dem Calcinieren die aktive Komponente des Katalysators zu 30 bis 60 Gew.-% aus Zinkoxid und zu 40 bis 70 Gew.-% aus Calciumcarbonat, welches bevorzugt in der Calcit-Modifikation vorliegt, besteht.

Als Base verwendet man wasserlösliche basische Carbonate wie Alkalimetallcarbonate wie Natriumcarbonat, Kaliumcarbonat, Alkalimetallhydrogencarbonate wie Natriumhydrogencarbonat, Kaliumhydrogencarbonat, Ammoniumcarbonat oder Ammoniumhydrogencarbonat sowie deren Mischungen, vorzugsweise Natriumcarbonat, besonders bevorzugt in Form ihrer wässrigen Lösungen in Konzentrationen im allgemeinen im Bereich von 0,5 bis 30, bevorzugt von 10 bis 25 Gramm Base/100 Gramm Lösung.

Die Fällung führt man im allgemeinen bei Temperaturen im Bereich von 10 bis 90°C, vorzugsweise von 40 bis 80°C durch. Nach dem Ausfällen kann man gewünschtenfalls den Niederschlag abfiltrieren. Der gewünschtenfalls abfiltrierte Niederschlag wäscht man in der Regel mit Wasser, bevorzugt so lange, bis kein Nitrat mehr mittels der Nitratringprobe feststellbar ist, und trocknet ihn anschließend bevorzugt bei einer Temperatur im Bereich von 90 bis 150°C unter Erhalt eines getrockneten Pulvers. Die Trocknung kann in ruhender oder bewegter Schicht, vorzugsweise durch Sprühtrocknung, erfolgen.

Das getrocknete Pulver wird anschließend bei Temperaturen von nicht höher als 600°C, bevorzugt im Bereich von 300 bis 600°C, insbesondere von 400 bis 475°C, bevorzugt in Luft, calciniert. Nach bisherigen Beobachtungen führt eine längere Erhitzung über 600°C zur Bildung der Aragonit-Modifikation von CaCO₃. Eine kurzfristige Erhitzung über 600°C ist dann nicht hinderlich für die Herstellung der erfindungsgemäß einsetzbaren Katalysatoren, solange sich dabei kein Aragonit (Nachweis mittels Röntgendiffraktometrie) bildet.

Nach dem Calcinieren kann man gewünschtenfalls das calcinierte Pulver zu Formkörpern wie Tabletten, Ringe, Zylinder etc., bevorzugt Tabletten, verpressen.

In einer bevorzugten Ausführungsform verpresst man das calcinierte Pulver zusammen mit Graphit, bevorzugt mit 0,1 bis 5, besonders bevorzugt mit 1 bis 2,5, insbesondere 2 Gew.%, bezogen auf die Gesamtmasse, Graphit.

In einer weiteren bevorzugten Ausführungsform verpresst man das uncalcinierte Pulver aus Schritt III. (s.o.) zu Formkörpern, bevorzugt zu Tabletten, Ringtabletten, kalottierten Tabletten, wie in der US-6,518,220 beschrieben, oder Triloben und calciniert die so erhaltenen Formkörper wie vorstehend beschrieben. Alternativ kann auch eine Extrusion zu Strängen oder Sternsträngen, bevorzugt zu Strängen, durchgeführt werden.

Die so erhaltenen calcinierten Pulver und Formkörper können als Katalysatoren eingesetzt werden, wobei diese Katalysatoren als aktive Komponenten Zinkoxid und Calciumcarbonat (in der Calcit-Modifikation) und als passive Komponente gewünschtenfalls Graphit enthalten können.

In einer weiterhin bevorzugten Ausführungsform setzt man einen Dehydrierungskatalysator ein, der ein Porenvolumen im Bereich von 0,10 bis 0,50, insbesondere von 0,20 bis 0,35 cm³/g, bei einem Porendurchmesser im Bereich von 5 nm bis 300 mm aufweist, wobei besonders bevorzugt mindestens 85 %, vorzugsweise mehr als 90 % dieses Porenvolumens mit einem Porendurchmesser im Bereich von 0,01 bis 0,5 mm verbunden ist.

Besonders bevorzugte Katalysatoren der genannten Art sind solche, die eine Stirndruckfestigkeit im Bereich von 500 bis 4000 N/cm², insbesondere von 1000 bis 2500 N/cm² und eine Seitendruckfestigkeit von 30 bis 300 N, vorzugsweise 50 bis 200 N aufweisen.

Die spezifische Oberfläche nach BET beträgt im allgemeinen 5 bis 50 m²/g, vorzugsweise 10 bis 30 m²/g. Das Porenvolumen im Porendurchmesserbereich zwischen 0,1 nm und 300 nm besitzt Werte üblicherweise zwischen 0,1 und 0,5 cm³/g, vorzugsweise 0,2 bis 0,35 cm³/g mit der Maßgabe, dass mindestens 85 %, vorzugsweise mehr als 90 % dieses Porenvolumens im Porendurchmesserbereich von 0,01 bis 0,5 mm liegen.

Die Stirndruckfestigkeit der Tabletten beträgt vorzugsweise 500 bis 4000 N/cm², insbesondere 1000 bis 2500 N/cm² und die Seitendruckfestigkeit der Pillen liegt bevorzugt zwischen 30 und 300 N, vorzugsweise 50 bis 200 N.

In einer besonders bevorzugten Ausführungsform wäscht man den Niederschlag aus schwerlöslichen Zink- und Calciumverbindungen, bevorzugt Zinkhydroxidcarbonat und Calciumcarbonat, auf Filterpressen, maischt den dabei erhaltenen Filterkuchen mit Wasser an, und versprüht die Maische zum Trocknen in einem Sprühturm. Das auf diese Weise erhaltene getrocknete Sprühpulver kann man danach wie oben beschrieben weiterverarbeiten.

Im Rahmen der bevorzugten Ausführungsform von Schritt b) des erfindungsgemäßen Gesamtverfahrens bringt man das verdampfte, gasförmige, optisch aktive Citronellol in an sich üblicher Weise in Kontakt mit dem eingesetzten Dehydrierkatalysator, beispielsweise in einem Festbettreaktor, Rohrreaktor, Rohrbündelreaktor oder in einem Wirbelschichtreaktor, bevorzugt in einem Rohrreaktor, in dem der Katalysator fest angeordnet ist. Besonders bevorzugt sind Rohrbündelreaktoren. Der Austrag wird üblicherweise destillativ aufgearbeitet.

Im allgemeinen verdampft man das eingesetzte optisch aktive Citronellol in an sich bekannter Weise, beispielsweise in einem geeigneten Verdampfer.

Das im Rahmen der bevorzugten Ausführungsform vorgesehen Dehydrierverfahren führt man üblicherweise bei erhöhter Temperatur durch. Die Temperatur der Gasphase in der Reaktionszone wählt man üblicherweise im Bereich von 350 bis 450°C. Den Druck der Gasphase in der Reaktionszone wählt man im allgemeinen im Bereich von 0,3 bis 10 bar.

Die Belastung des Katalysators wählt man im allgemeinen im Bereich von 0,5 bis 3,0, vorzugsweise von 0,6 bis 2,0 Liter optisch aktives Citronellol pro Liter an Katalysator und pro Stunde.

Geeignete Reaktorformen sind der Festbettrohr- oder Rohrbündelreaktor. Bei diesen befindet sich der gewählte Katalysator als Festbett in einem Reaktionsrohr oder in einem Bündel von Reaktionsrohren. Die Reaktionsrohre werden üblicherweise dadurch indirekt beheizt, dass in dem die Reaktionsrohre umgebenden Raum ein Gas, z.B. ein Kohlenwasserstoff wie Methan, verbrannt wird oder ein Wärmeträgermedium (Salzbad, Wälzgas etc.) eingesetzt wird. Es kann auch eine elektrische Beheizung der Reaktionsrohre mit Heizmanschetten erfolgen. Übliche Reaktionsrohr-Innendurchmesser betragen etwa 2,5 bis 15 cm. Ein typischer Dehydrierrohrbündelreaktor umfasst ca. 10 bis 32000 Reaktionsrohre, bevorzugt ca. 10 bis 200 Reaktionsrohre. Die Temperatur im Reaktionsrohrinneren bewegt sich üblicherweise im Bereich von 250 bis 600°C, vorzugsweise im Bereich von 300 bis 600°C. Der Arbeitsdruck liegt üblicherweise zwischen 0,5 und 8 bar, häufig zwischen 1 und 2 bar.

Das bevorzugte Dehydrierverfahren kann auch, wie in Chem. Eng. Sci. 1992 b, 47 (9-11) 2313 beschrieben, heterogen katalysiert im Wirbelbett durchgeführt werden. Zweckmäßigerweise werden dabei zwei Wirbelbetten nebeneinander betrieben, von denen sich eines in der Regel im Zustand der Regenerierung befindet. Der Arbeitsdruck beträgt typischerweise 1 bis 2 bar, die Dehydriertemperatur in der Regel 250 bis 600°C.

Die erfindungsgemäß bevorzugte katalytische Dehydrierung kann mit oder ohne sauerstoffhaltigem Gas als Co-Feed und optional unter Zusatz von Wasserdampf, Stickstoff, Methan und/oder Argon durchgeführt werden. Der gewählte Reaktor kann ein oder mehrere aufeinanderfolgende Katalysatorbetten aufweisen. Die Anzahl der Katalysatorbetten kann 1 bis 20, zweckmäßigerweise 1 bis 6, bevorzugt 1 bis 4 und insbesondere 1 bis 3 betragen. Die Katalysatorbetten werden vorzugsweise radial oder axial vom Reaktionsgas durchströmt. Im Allgemeinen wird ein solcher Hordenreaktor mit einem Katalysatorfestbett betrieben. Im einfachsten Fall sind die Katalysatorfestbetten in einem Schachtofenreaktor axial oder in den Ringspalten von konzentrisch angeordneten zylindrischen Gitterrosten angeordnet. Ein Schachtofenreaktor entspricht einer Horde.

In einer besonders bevorzugten Ausführungsform des erfindungsgemäß bevorzugten Dehydrierverfahrens gemäß Schritt b) setzt man optisch aktives Citronellol mit einem Enantiomerenüberschuss von mindestens 90% ee, bevorzugt 95% ee an einem Katalysator, dessen Aktivkomponenete 54 bis 57 Gew.-% Zinkoxid und 43 bis 46 Gew.-% Calciumcarbonat enthält (jeweils bestimmt in Form der Oxide der geglühten Katalysatormasse) in einem geeigneten Reaktor, beispielsweise einem Rohrreaktor um. Der Reaktor kann dabei durch jede geeignete Methode, bevorzugt durch eine Salzschmelze, auf Temperaturen im Bereich von etwa 350 bis etwa 450°C beheizt werden. Die Reaktion findet in der Gasphase statt. Gute Resultate erhält man insbesondere, wenn die Reaktion in Abwesenheit von Sauerstoff durchgeführt wird. Dazu leitet man ein das zu dehydrierende Edukt enthaltende Stoffgemisch beispielsweise in einem Inertgasstrom wie z.B. einem Stickstoffstrom über den gewählten Katalysator. Optional ist auch eine autotherme Fahrweise durch partielle H₂-Verbrennung nach vorheriger Einspeisung eines H₂-haltigen Stoffgemisches möglich.

Die Isolierung der Reaktionsprodukte kann nach allen geeigneten und dem Fachmann an sich bekannten Methoden vorgenommen werden. Man erhält auf diese Weise bei einem Umsatz von vorzugsweise etwa 30 bis etwa 60% d. Th. optisch aktives Citronallal in einer Selektivität von normalerweise etwa 60 bis etwa 95%.

### Beschreibung Schritt c): Cyclisierung

Das gemäß Schritt b) des erfindungsgemäßen Verfahrens erhaltene optisch aktive Citronellal wird erfindungsgemäß zu einem optisch aktives Isopulegol der Formel (IX) 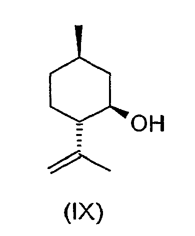 bzw. in Abhängigkeit vom eingesetzten Enantiomeren des Citronellals dessen Enantiomeren ent-(IX) enthaltenden Stoffgemisch cyclisiert. Bei der Cyclisierung von Citronellal zu Isopulegol fallen, in Abhängigkeit von den gewählten Reaktionsbedingungen und den gewählten Cyclisierungskatalysatoren, in der Regel Gemische der vier möglichen Diastereomere des Isopulegols (Isopulegol, Neo-Isopulegol, Iso-Isopulegol und Neoiso-Isopulegol) an. Bei Einsatz von optisch aktivem Citronallal erhält man Gemische der vier Diastereomere in optisch aktiver Form.

Die Cyclisierung gemäß Schritt c) des erfindungsgemäßen Verfahrens wird üblicherweise in Gegenwart einer Säure oder Lewis-Säure als Cyclisierungskatalysator durchgeführt wie beispielsweise von Y. Nakatani und K. Kawashima unter Einsatz von Zinkhalogeniden in Synthesis, 1978, 147-148 und den darin erwähnten Zitaten beschrieben. Auch die thermische Cyclisierung mit allerdings moderaten Stereoselektivitäten ist möglich (K.H. Schulte-Elte et al. Helv. Chim. Acta, 1976, 50, 153-165).

Brauchbare Cyclisierungskatalysatoren sind beispielsweise ZnBr₂, wie beispielsweise in der JP-A 53116348 offenbart, Scandiumtriflat, wie in der EP-A 0 926 117 beschrieben, SiO₂ (Kieselgel), wie in der US 2,117,414 beschrieben, Mischoxidkatalysatoren wie beispielsweise SiO₂-Al₂O₃, Montmorillonit, Alumosilicate, Eisen(III)chlorid, Zinkchlorid, Zinntetrachlorid und Trisdiarylphenoxyaluminium-Komplexe, wie aus der EP-A 1 225 163 bekannt oder auch Zeolithe.

Die Cyclisierung von optisch aktivem Citronellal zu Gemischen, die optisch aktives Isopulegol enthalten kann unter einer breiten Vielfalt von Reaktionsbedingungen durchgeführt werden. Insbesondere die Wahl eines geeigneten Lösungsmittels, die Konzentration des Substrats im Reaktionsgemische sowie Reaktionszeit und -temperatur können breit variiert werden. Darüber hinaus können neben thermischer Energie auch andere Energieformen, insbesonder Mikrowellenenergie oder Ultraschall zugeführt werden. Der Fachmann wählt die Reaktionsbedingungen bevorzugt so aus, dass er durch die Cyclisierung des optisch aktiven Citronellals gemäss Schritt c) des erfindungsgemäßen Verfahrens (nach Entfernen der Lösungsmittel) ein Stoffgemisch erhält, das zu mindestens 60 Gew.-%, bevorzugt zu mindestens 75 Gew-% aus Isopulegol besteht.

Die gemäß Schritt c) erhaltenen Stoffgemische enthalten neben dem gewünschten optisch aktiven Isopulegeol in der Regel auch die drei weiteren, vorstehend genannten Diasteromere des Isopulegols in veränderlichen Anteilen sowie geringe Mengen weiterer Verunreinigungen bzw. Lösemittelreste.

### Beschreibung Schritt d): Trennung und Hydrierung

Die so erhaltenen Stoffgemische werden gemäß Schritt d) des erfindungsgemäßen Verfahrens einer Trennung sowie einer Hydrierung unterzogen, wobei man entweder optisch aktives Isopulegol aus dem so erhaltenen Stoffgemisch abtrennt und zu optisch aktivem Menthol hydriert oder das in dem so erhaltenen Stoffgemisch enthaltene optisch aktiven Isopulegeol zu optisch aktivem Menthol hydriert und das so erhaltene optisch aktive Menthol aus dem als Produkt der Hydrierung erhaltenem Stoffgemisch abtrennt.

Erfindungsgemäß lassen sich die gemäß Schritt c) erhaltenen Stoffgemische im Rahmen einer ersten Alternative einer Trennung unterziehen, bei der man das im Stoffgemisch enthaltene optisch aktive Isopulegol von den restlichen Komponenten des Stoffgemisches, insbesondere von den drei weiteren Diastereomeren des Isopulegols, abtrennt.

Zur Trennung gemäß Schritt d) des erfindungsgemäßen Verfahren eigenen sich alle dem Fachmann geeignet erscheinenden Methoden der Trennung von Stoffgemischen, insbesondere der Trennung von Diastereomerengemischen. Geeignete Verfahren zur Abtrennung des optisch aktiven Isopulegols umfassen beispielsweise Kristallisationsverfahren, gegebenenfalls nach vorheriger Derivatisierung der erhaltenen Isopulegol-Diastereomere, Destillationsverfahren und chromatographische Verfahren. Im Rahmen der vorliegenden Erfindung bevorzugte Trennverfahren zur Abtrennung des erfindungsgemäß erhaltenen optisch aktiven Isopulegols ist die Destillation oder die Kristallisiation. Eine Erhöhung der optischen Reinheit des erfindungsgemäß erhaltenen Isopulegols auf > 99.7% ee ist beispielsweise durch Kristallisation bei tiefen Temperaturen, z.B. bei -40 °C möglich, wie in der US 5,663,460 beschreiben.

Das gemäß dieser ersten Alternative gemäß Schritt d) des erfindungsgemäßen Verfahrens abgetrennte optisch aktive Isopulegol wird anschließend zu optisch aktivem Menthol hydriert.

Gemäß der zweiten Alternative von Schritt d) des erfindungsgemäßen Verfahrens lassen sich die gemäß schritt c) erhaltenen Stoffgemische auch zunächst einer Hydrierung unterwerfen, wobei man das in dem Stoffgemisch enthaltene optisch aktive Isopulegol zu optisch aktivem Menthol hydriert und das so erhaltene optisch aktive Menthol aus dem durch Hydrierung erhaltenen Produktgemisch abtrennt.

Zur Trennung des so erhaltenen optisch aktiven Menthols von den gegebenfalls daneben erhaltenen weiteren Diasteromeren und/oder Verunreinigung eignen sich die vorstehend zur Abtrennung von optisch aktivem Isopulegol genannten Trennverfahren.

Die in beiden Alternativen durchzuführende Hydrierung der ethylenischen Doppelbindung des optisch aktiven Isopulegols bzw. dessen Diastereomerer eignen sich alle dem Fachmann geeignet erscheinenden Hydrierverfahren, insbesondere Verfahren zur katalytischen Hydrierung ethylenischer Doppelbindungen an geeigneten Übergangsmetall-haltigen Katalysatoren in Gegenwart von Wasserstoff oder Wasserstoffdonatoren. Dabei sind homogen- und heterogenkatalytische Verfahren gleichermaßen geeignet, wobei bei Umsetzungen im technischen Maßstab heterogenkatalytische Methoden zu bevorzugen sind. Als Katalysatoren eignen sich insbesondere solche, die mindestens ein Übergangsmetall der Gruppen 7 bis 9 des Periodensystems der Elemente, insbesondere Pd, Pt, Ni, Ir, Rh und/oder Ru enthalten. Geeignete heterogene Katalysatoren können in geträgerter wie ungeträgerter Form eingesetzt werden. Mögliche Ausgestaltungen, Verfahrensvarianten bzw. Katalysatoren gehören zum Wissen des Fachmannes.

Die Hydrierung von Isopulegol mit Pd-Suspensionskatalysatoren ist beispielsweise beschrieben in der EP-A 1 053 974. Die Hydrierung verschiedener cyclischer Monoterpenderivate zu Menthol ist auch in WO 2004/013339 offenbart.

Bevorzugt führt man Schritt d) des erfindungsgemäßen Verfahrens so durch, dass man zunächst das gemäß Schritt c) erhaltenene Stoffgemisch hydriert und anschließend optisch aktives Menthol aus dem durch Hydrierung erhaltenen Stoffgemisch abtrennt. Insbesondere bevorzugt trennt man das durch Hydrierung erhaltene optisch aktive Menthol durch Kristallisation von den weiteren Komponenten des durch Hydrierung erhaltenen Stoffgemisches ab.

In einer besonders bevorzugten Ausführungsform des erfindungsgemäßen Verfahrens führt man gemäß Schritt a) des erfindungsgemäßen Verfahrens eine enantioselektive Hydrierung von Geraniol der Formel (1), bevorzugt solchem, das zu etwa 0,1 bis etwa 5 Gew.-% Neral enthält zu D-(R)-Citronellol der Formel (X) durch. Bevorzugt setzt man dabei einen Ru und (S)-BINAP enthaltenden Katalysator ein und erhält bevorzugt D-(R)-Citronellol mit einer Reinheit von 90 bis 99% ee. Das so erhaltene D-(R)-Citronellol wird dann im Rahmen der besonders bevorzugten Ausführungsform durch Dehydrierung in D-(R)-Citronellal der Formel (XI) überführt, wobei man insbesondere bevorzugt eine wie vorstehend beschriebene Dehydrierung in der Gasphase durchführt. Das so erhaltene D-(R)-Citronellal wird anschließend im Rahmen der bevorzugten Ausführungsform in Gegenwart von ZnBr₂ oder Al₂O₃/SiO₂ als Katalysator zu L-Isopulegol der Formel (IX) cyclisiert. Das durch Cyclisierung erhaltene L-Isopulegol wird anschließend wie vorstehend beschrieben zum L-Menthol der Formel (XII) hydriert und abschließend durch ein geeignetes Trennverfahren, insbesondere durch Kristallisation abgetrennt.

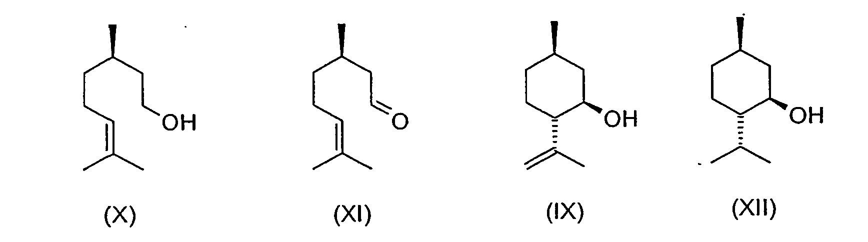

Das erfindungsgemäße Verfahren zeichnet sich durch die Kombination der Einzelschritte a) bis d) aus. Diese Kombination eröffnet einen vorteilhaften, in technischem Maßstab gut durchführbaren und zu kommerziell akzeptablen Gesamtausbeuten führenden Zugang zu optisch aktivem Menthol. Dabei ist von besonderer Bedeutung, dass der offenbarte Syntheseweg unabhängig ist von aus natürlichen Quellen zu isolierenden Ausgangsstoffen oder Zwischenprodukten, die, falls chiral, in der Regel nur in Form eines ihrer Enantiomeren verfügbar sind. Als weiteren Vorteil eröffnet das erfindungsgemäße Verfahren daher die Möglichkeit, wahlweise beide Enantiomere des Menthols in technischem Maßstab bereitzustellen.

### Beispiele:

Die folgenden Beispiele dienen der Erläuterung der Erfindung, ohne sie jedoch in irgendeiner Weise zu beschränken:

### Beispiel 1: Enantioselektive Hydrierung von Geraniol mit dem Katalysatorsystem Ru/(S)-BINAP

Unter Inertgasatmosphäre wurden 2,4 mg [RuCl₂(C₆H₆)]₂ (entspr. 0,01 µmol Ru) in 75 ml Methanol gelöst, 6,0 mg 80,01 µmol (s)-BINAP zugegeben und 12 h bei Raumtemperatur gerührt, bis eine klare Lösung resultierte. Die Katalysatorlösung wurde in einen Autoklaven mit Begasungsrührer überführt und weitere 75 ml Methanol sowie 14.8 g Geraniol (0,096 mol, Reinheit 95%) zugegeben. Bei einem Wasserstoffdruck von 100 bar und einer Temperatur von 40°C wurde 24 h gerührt. Danach wurde gaschromatographisch ein Umsatz von 98,3% bestimmt. Man erhielt eine Ausbeute von 97,2 % (R)-Citronellol mit einer Enantiomerenreinheit von 95,2% ee.

### Beispiel 2: Dehydrierung von optisch aktivem Citronellol in der Gasphase

Ein durch eine Salzschmelze beheizbarer Rohrreaktor wurde mit 10,8 g eines Katalysators bestehend aus 55 Gew.-% ZnO, und 45 Gew.-% CaCO₃ in der Calcit-Modifikation (jeweils bestimmt in Form der Oxide der geglühten Katalysatormasse) beschickt. Bei einer Temperatur von 400°C wurden ein Gemisch von 46 NI/h Stickstoff und 3,44 g/h R-Citronellol mit einem Enantiomerenüberschuss von 95% ee über die Schüttung geleitet. Man erhielt bei einem Umsatz von 50,2 % in einer Selektivität von 75,5% Citronellal mit einem Enantiomerenüberschuss bezüglich R-Citronellal von 95% ee.

## Patentansprüche

1. Verfahren zur Herstellung von optisch aktivem Menthol ausgehend von Geraniol oder Nerol oder Gemischen von Geraniol und Nerol indem man
a) Geraniol oder Nerol oder Gemische von Geraniol und Nerol zu optisch aktivem Citronellol enantioselektiv hydriert,
b) das so erhaltene optisch aktive Citronellol zu optisch aktivem Citronellal umsetzt,
c) das so erhaltene optisch aktive Citronellal zu einem optisch aktives Isopulegol enthaltenden Stoffgemisch cyclisiert und
d) optisch aktives Isopulegol aus dem so erhaltenen Stoffgemisch abtrennt und zu optisch aktivem Menthol hydriert oder das in dem so erhaltenen Stoffgemisch enthaltene optisch aktive Isopulegol zu optisch aktivem Menthol hydriert und das so erhaltene optisch aktive Menthol aus dem als Produkt der Hydrierung erhaltenen Stoffgemisch abtrennt.

2. Verfahren nach Anspruch 1, **dadurch gekennzeichnet, dass** man als Ausgangsstoff Geraniol einsetzt.

3. Verfahren nach Anspruch 1 oder 2, **dadurch gekennzeichnet, dass** man die enantioselektive Hydrierung gemäß Schritt a) in Gegenwart eines homogenen Übergangsmetallkatalysators durchführt, der Ru, Rh oder Ir sowie einen chiralen, mindestens ein Phosphor-Atom aufweisenden Liganden enthält.

4. Verfahren nach Anspruch 3, **dadurch gekennzeichnet, dass** der Übergangsmetallkatalysator Ru, Rh oder Ir und einen Liganden enthält, der die Fähigkeit aufweist Atropisomerie bezüglich zweier Aryl- bzw. Hetarylsysteme auszubilden.

5. Verfahren nach Anspruch 3 oder 4, **dadurch gekennzeichnet, dass** der Übergangsmetallkatalysator Ru und einen Liganden ausgewählt aus der Gruppe der Liganden 2,2'-Bis(diphenylphosphino-1,1'-binaphtyl, 2,2'-Bis[di(p-tolyl)-phosphino]-1,1'-binaphthyl, 2,2'-Bis(diphenylphosphino)-4,4',6,6'-tetramethyl-3,3'-bibenzo[b]thiophen und 2,2'-Bis(diphenylphosphino)-3,3'-tetramethyl-3,3'-bibenzo[b]thiophen enthält.

6. Verfahren nach einem der Ansprüche 2 bis 5, **dadurch gekennzeichnet, dass** man zur Durchführung der enantioselektiven Hydrierung gemäß Schritt a) Methanol als Lösemittel einsetzt.

7. Verfahren nach Anspruch 6, **dadurch gekennzeichnet, dass** man eine 3 bis 20 Gew.%ige Lösung von Geraniol in Methanol einsetzt.

8. Verfahren nach einem der Ansprüche 1 bis 7, **dadurch gekennzeichnet, dass** man gemäß Schritt b) das optisch aktive Citronellol zu optisch aktivem Citronellal umsetzt, indem man eine Dehydrierung in Gegenwart eines Katalysators in der Gasphase durchführt.

9. Verfahren nach Anspruch 8, **dadurch gekennzeichnet, dass** man einen Katalysator einsetzt, der Zinkoxid und Calciumcarbonat enthält.

10. Verfahren nach Anspruch 8 oder 9, **dadurch gekennzeichnet, dass** man einen Katalysator einsetzt, dessen aktive Komponente zu 30 bis 60 Gew.-% aus Zinkoxid und zur 40 bis 70 Gew.-% aus Calciumcarbonat besteht.

11. Verfahren nach einem der Ansprüche 1 bis 10, **dadurch gekennzeichnet, dass** man die Cyclisierung gemäß Schritt c) in Gegenwart einer Säure oder Lewis-Säure durchführt.

12. Verfahren nach Anspruch 11, **dadurch gekennzeichnet, dass** man als Säure oder Lewis-Säure ZnBr₂, Scandiumtriflat, SiO₂, SiO₂-Al₂O₃, Montmorillonit, Alumosilicate, Eisen(III)chlorid, Zinkchlorid, Zinntetrachlorid, Trisdiarylphenoxyaluminium-Komplexe und/oder Zeolithe einsetzt.

13. Verfahren nach einem der Ansprüche 1 bis 12, **dadurch gekennzeichnet, dass** man zur Abtrennung des Isopulegols gemäss Schritt d) eine Destillation und/oder eine Kristallisation durchführt.

14. Verfahren nach einem der Ansprüche 1 bis 13, **dadurch gekennzeichnet, dass** man gemäss Schritt d) eine katalytische Hydrierung von Isopulegol zu Menthol durchführt.

15. Verfahren nach einem der Ansprüche 1 bis 14, **dadurch gekennzeichnet, dass** man Geraniol gemäss Schritt a) zu D-(R)-Citronellol enantioselektiv hydriert.

16. Verfahren nach einem der Ansprüche 1 bis 15, **dadurch gekennzeichnet, dass** man D-(R)-Citronellol gemäss Schritt b) in D-(R)-Citronellal überführt.

17. Verfahren nach einem der Ansprüche 1 bis 16, zur Herstellung von L-Menthol.

## Claims

1. A process for the preparation of optically active menthol from geraniol or nerol or mixtures of geraniol and nerol by
a) enantioselectively hydrogenating geraniol or nerol or mixtures of geraniol and nerol to optically active citronellol,
b) converting the resulting optically active citronellol to optically active citronellal,
c) cyclizing the resulting optically active citronellal to a mixture of substances comprising optically active isopulegol, and
d) separating optically active isopulegol from the resulting mixture of substances and hydrogenating it to optically active menthol, or hydrogenating the optically active isopulegol present in the resulting mixture of substances to optically active menthol and separating the resulting optically active menthol from the mixture of substances obtained as the hydrogenation product.

2. The process according to claim 1 wherein geraniol is used as the starting material.

3. The process according to claim 1 or 2 wherein the enantioselective hydrogenation of step a) is carried out in the presence of a homogeneous transition metal catalyst comprising Ru, Rh or Ir and a chiral ligand containing at least one phosphorus atom.

4. The process according to claim 3 wherein the transition metal catalyst comprises Ru, Rh or Ir and a ligand which is capable of forming atropisomers in respect of two aryl or heteroaryl systems.

5. The process according to claim 3 or 4 wherein the transition metal catalyst comprises Ru and a ligand selected from the group comprising the ligands 2,2'-bis(diphenylphosphino)-1,1'-binaphthyl, 2,2'-bis[di (p-tolyl) phosphino]-1,1'-binaphthyl, 2,2'-bis(diphenylphosphino)-4,4',6,6'-tetramethyl-3,3'-bibenzo[b]thiophene and 2,2'-bis-(diphenylphosphino)-3,3'-tetramethyl-3,3'-bibenzo-[b] thiophene .

6. The process according to one of claims 2 to 5 wherein methanol is used as the solvent to carry out the enantioselective hydrogenation of step a).

7. The process according to claim 6 wherein a 3 to 20% by weight solution of geraniol in methanol is used.

8. The process according to one of claims 1 to 7 wherein, in step b), the optically active citronellol is converted to optically active citronellal by carrying out a dehydrogenation in the presence of a catalyst in the gas phase.

9. The process according to claim 8 wherein the catalyst used comprises zinc oxide and calcium carbonate.

10. The process according to claim 8 or 9 wherein a catalyst is used whose active component consists of 30 to 60% by weight of zinc oxide and 40 to 70% by weight of calcium carbonate.

11. The process according to one of claims 1 to 10 wherein the cyclization of step c) is carried out in the presence of an acid or Lewis acid.

12. The process according to claim 11 wherein the acid or Lewis acid used is ZnBr₂, scandium triflate, SiO₂, SiO₂-Al₂O₃, montmorillonite, aluminosilicates, iron(III) chloride, zinc chloride, tin tetrachloride, trisdiarylphenoxyaluminum complexes and/or zeolites.

13. The process according to one of claims 1 to 12 wherein the isopulegol is separated off in step d) by distillation and/or crystallization.

14. The process according to one of claims 1 to 13 wherein isopulegol is catalytically hydrogenated to menthol in step d).

15. The process according to one of claims 1 to 14 wherein geraniol is enantioselectively hydrogenated to D-(R)-citronellol in step a).

16. The process according to one of claims 1 to 15 wherein D-(R)-citronellol is converted to D-(R)-citronellal in step b).

17. The process according to one of claims 1 to 16 for the preparation of L-menthol.

## Revendications

1. Procédé de fabrication de menthol optiquement actif à partir de géraniol ou de nérol ou de mélanges de géraniol et de nérol, selon lequel
a) du géraniol ou du nérol ou des mélanges de géraniol et de nérol sont hydrogénés de manière énantiosélective en citronellol optiquement actif,
b) le citronellol optiquement actif ainsi obtenu est transformé en citronellal optiquement actif,
c) le citronellal optiquement actif ainsi obtenu est cyclisé en un mélange de substances contenant de l'isopulégol optiquement actif et
d) l'isopulégol optiquement actif est séparé du mélange de substances ainsi obtenu et hydrogéné en menthol optiquement actif ou l'isopulégol optiquement actif contenu dans le mélange de substances ainsi obtenu est hydrogéné en menthol optiquement actif et le menthol optiquement actif ainsi obtenu est séparé du mélange de substances obtenu en tant que produit de l'hydrogénation.

2. Procédé selon la revendication 1, **caractérisé en ce que** le géraniol est utilisé en tant que substance de départ.

3. Procédé selon la revendication 1 ou 2, **caractérisé en ce que** l'hydrogénation énantiosélective selon l'étape a) est réalisée en présence d'un catalyseur homogène à base de métaux de transition qui contient Ru, Rh ou Ir et un ligand chiral comportant au moins un atome de phosphore.

4. Procédé selon la revendication 3, **caractérisé en ce que** le catalyseur à base de métaux de transition contient Ru, Rh ou Ir et un ligand qui présente la capacité de développer une atropisomérie relative à deux systèmes aryle ou hétaryle.

5. Procédé selon la revendication 3 ou 4, **caractérisé en ce que** le catalyseur à base de métaux de transition contient Ru et un ligand choisi dans le groupe des ligands 2,2'-bis(diphénylphosphino)-1,1'-binaphtyle, 2,2'-bis[di(p-tolyl)-phosphino]-1,1'-binaphtyle, 2,2'-bis(diphénylphosphino)-4,4',6,6'-tétraméthyl-3,3'-bibenzo[b]thiophène et 2,2'-bis (diphénylphosphino)-3,3'-tétraméthyl-3,3'-bibenzo[b]thiophène.

6. Procédé selon l'une quelconque des revendications 2 à 5, **caractérisé en ce que** le méthanol est utilisé en tant que solvant pour réaliser l'hydrogénation énantiosélective selon l'étape a).

7. Procédé selon la revendication 6, **caractérisé en ce qu'**une solution à 3 à 20 % en poids de géraniol dans du méthanol est utilisée.

8. Procédé selon l'une quelconque des revendications 1 à 7, **caractérisé en ce que** selon l'étape b), le citronellol optiquement actif est transformé en citronellal optiquement actif en réalisant une déshydrogénation en présence d'un catalyseur dans la phase gazeuse.

9. Procédé selon la revendication 8, **caractérisé en ce qu'**un catalyseur qui contient de l'oxyde de zinc et du carbonate de calcium est utilisé.

10. Procédé selon la revendication 8 ou 9, **caractérisé en ce qu'**un catalyseur dont le composant actif est constitué de 30 à 60 % en poids d'oxyde de zinc et de 40 à 70 % en poids de carbonate de calcium est utilisé.

11. Procédé selon l'une quelconque des revendications 1 à 10, **caractérisé en ce que** la cyclisation selon l'étape c) est réalisée en présence d'un acide ou d'un acide de Lewis.

12. Procédé selon la revendication 11, **caractérisé en ce que** du ZnBr₂, du triflate de scandium, du SiO₂, du Si₂-A1₂O₃, de la montmorillonite, des alumosilicates, du chlorure de fer (III), du chlorure de zinc, du tétrachlorure d'étain, des complexes de trisdiarylphénoxyaluminium et/ou des zéolithes sont utilisés en tant qu'acide ou acide de Lewis.

13. Procédé selon l'une quelconque des revendications 1 à 12, **caractérisé en ce qu'**une distillation et/ou une cristallisation sont réalisées pour la séparation de l'isopulégol selon l'étape d).

14. Procédé selon l'une quelconque des revendications 1 à 13, **caractérisé en ce que** selon l'étape d), une hydrogénation catalytique d'isopulégol en menthol est réalisée.

15. Procédé selon l'une quelconque des revendications 1 à 14, **caractérisé en ce que** le géraniol est hydrogéné de manière énantiosélective en D-(R)-citronellol selon l'étape a).

16. Procédé selon l'une quelconque des revendications 1 à 15, **caractérisé en ce que** le D-(R)-citronellol est transformé en D-(R)-citronellal selon l'étape b).

17. Procédé selon l'une quelconque des revendications 1 à 16, pour la fabrication de L-menthol.
